# EUROPÄISCHE PATENTSCHRIFT

(11) **EP 3 409 187 B1**
(45) Veröffentlichungstag und Bekanntmachung des Hinweises auf die Patenterteilung: **16.08.2023**
(21) Anmeldenummer: 17173406.4
(22) Anmeldetag: 30.05.2017
(51) Int. Cl.: A61B 5/02, A61B 5/021, A61B 5/00

(54) **VERFAHREN UND VORRICHTUNG ZUR BESTIMMUNG EINER STRÖMUNGSSITUATION IN EINEM ORGAN**
METHOD AND DEVICE FOR DETERMINING A FLOW SITUATION IN AN ORGAN
PROCÉDÉ ET DISPOSITIF DE DÉTERMINATION D'UNE SITUATION D'ÉCOULEMENT DANS UN ORGAN

(43) Veröffentlichungstag der Anmeldung: 05.12.2018
(73) Patentinhaber: Siemens Healthcare GmbH, 91052 Erlangen (DE)
(72) Erfinder: Schmidt, Sebastian, 91085 Weisendorf (DE)

(56) Entgegenhaltungen:
- EDUARD ROHAN ET AL: "Modeling of the contrast-enhanced perfusion test in liver based on the multi-compartment flow in porous media", ARXIV.ORG, CORNELL UNIVERSITY LIBRARY, 201 OLIN LIBRARY CORNELL UNIVERSITY ITHACA, NY 14853, 30. Mai 2016 (2016-05-30), XP080704439,
- VLADIMÍR LUKES ET AL: "Numerical simulation of liver perfusion: from CT scans to FE model", 7TH EUROPEAN CONFERENCE ON PYTHON IN SCIENCE (EUROSCIPY 2014), 19. Dezember 2014 (2014-12-19), Seiten 79-84, XP055411748,

## Beschreibung

Die Erfindung betrifft ein Verfahren, das zur Bestimmung einer Strömungssituation in einem Gefäß, insbesondere in einem Blutgefäß dient. Die Erfindung betrifft des Weiteren eine Vorrichtung zur Bestimmung der Strömungssituation, insbesondere zur Durchführung des vorgenannten Verfahrens.

Eine Strömungssituation wird üblicherweise durch verschiedene Kenngrößen (Parameter) beschrieben, die für das strömende Fluid bestimmt werden können. Diese Kenngrößen geben dabei insbesondere den Strömungswiderstand innerhalb eines Gefäßes, den Druck des Fluids (insbesondere an einer bestimmten Stelle des Gefäßes), eine Flussrate (auch als Volumenstrom bezeichnet) und/oder eine Strömungsgeschwindigkeit wieder. Im medizinischen Bereich ist die Bestimmung der Strömungssituation in einem Blutgefäß häufig von hoher Wichtigkeit. Ein hoher Gefäßinnendruck, insbesondere Bluthochdruck (auch als "Hypertonie" bezeichnet), kann zwar auch durch vorübergehende Zustände (bspw. eine Krankheit oder eine Überlastung) hervorgerufen sein und dabei auch nur zeitlich vergleichsweise kurz vorherrschen. Bei andauerndem Vorliegen des hohen Gefäßinnendrucks (bspw. bei chronischen Krankheiten und/oder Gewebeveränderungen) kann dies allerdings zu einer Schädigung des Gefäßsystems und/oder des Herzens selbst führen. Zur Ermittlung insbesondere des globalen Blutdrucks eines Patienten - konkret des aktuellen Werts des Gefäßinnendrucks - steht als gängiges, insbesondere nicht-invasives Verfahren die Blutdruckmessung nach Riva-Rocci zur Verfügung. Dabei wird meist an einer Oberarmarterie mittels einer Druckmanschette und einem Stethoskop der systolische und diastolische Wert des Blutdrucks bestimmt. Der auf diese Weise bestimmte Blutdruck wird üblicherweise auch als arterieller Blutdruck bezeichnet.

Allerdings kann bei diversen Krankheiten ein erhöhter Gefäßinnendruck auch nur lokal in Körperregionen vorliegen, die für die vorstehend beschriebene Blutdruckmessung nicht zugänglich sind. Beispiele hierfür sind die sogenannte pulmonale Hypertonie (d. h. Bluthochdruck im Lungenkreislauf) und die sogenannte portale Hypertonie (d. h. Bluthochdruck in der Pfortader, insbesondere der Pfortader der Leber). Beide Zustände können dabei lebensbedrohlich für den Patienten sein.

Bei der pulmonalen Hypertonie ist regelmäßig der Strömungswiderstand der Blutgefäße der Lunge bspw. aufgrund von Veränderungen des Lungengewebes, insbesondere der Lungenbläschen (Alveolen) erhöht. Als Folge kann eine Überlastung der rechten Herzkammer auftreten, die langfristig auch zum Tode führen kann. Soweit anwendbar, können zur Therapie bspw. den Strömungswiderstand erhöhende Blutgerinnsel oder dergleichen operativ entfernt werden. Ursache für die portale Hypertonie ist meist eine Veränderung des Lebergewebes, bspw. eine Zirrhose. Als körpereigene "Reaktion" wird in diesem Fall teilweise Blut über andere Gefäße "umgeleitet", (bspw. über das venöse System des Ösophagus), wodurch sich dort jedoch Krampfadern bilden können, die das Risiko einer lebensbedrohlichen (inneren) Blutung erhöhen. Zur Therapie kann bspw. ein sogenannter porto-systemischer Shunt (eine Art "Kurzschluss") eingesetzt werden, mittels dessen das Blut an der Leber vorbei in die Hohlvene geleitet wird.

Neben den vorstehend beschriebenen, im Bereich der Kapillaren des zugeordneten Gefäßes (d. h. insbesondere innerhalb des Gewebebereichs des jeweiligen Organs) zu lokalisierenden Gründen für die Hypertonie, konkret für einen erhöhten Strömungswiderstand innerhalb des Gefäßes (bspw. aufgrund einer Leberzirrhose, eines Lungenemphysems, einer Lungenfibrose etc.), kann in beiden vorstehend beschriebenen Fällen der Strömungswiderstand aber auch aus innerhalb der vergleichsweise größeren Gefäße liegenden Gründen (bspw. durch eine Pfortaderthrombose, eine chronische thromboembolische Hypertonie, ein Budd-Chiari-Syndrom oder dergleichen) erhöht sein.

Um den Druck an derartigen, von der Körperaußenseite unzugänglichen Körperregionen, konkret Gefäßen ermitteln zu können, wird heutzutage meist auf invasive Methoden zurückgegriffen. Beispielsweise wird dabei ein Katheter in das Gefäß, in dem der Gefäßinnendruck zu bestimmen ist, eingebracht. Ein solcher Katheter trägt dabei einen Drucksensor und kann somit lokal den vorherrschenden Gefäßinnendruck insbesondere direkt messen. Alternativ kann auch auf das entsprechende Gefäß von der Gefäßaußenseite her mittels einer hinreichend nahe an das Gefäß zugeführten Sonde - bspw. mittels eines Gastroskops - Druck aufgebracht und somit der Gefäßinnendruck lokal bestimmt werden. Anerkanntermaßen ist hierbei jedoch stets ein Eingriff erforderlich, der in manchen Fällen unerwünscht sein kann.

Aus EDUARD ROHAN ET AL: "Modeling of the contrast-enhanced perfusion test in liver based on the multi-compartment flow in porous media", ARXIV.ORG, CORNELL UNIVERSITY LIBRARY, 201 OLIN LIBRARY CORNELL UNIVERSITY ITHACA, NY 14853, 30. Mai 2016 sowie aus VLADIMiR LUKES ET AL: "Numerical simulation of liver perfusion: from CT scans to FE model", 7TH EUROPEAN CONFERENCE ON PYTHON IN SCIENCE (EUROSCIPY 2014), 19. Dezember 2014, S. 79-84, ist ein Verfahren bekannt, bei der auf Basis von Daten einer medizinischen Bildgebung ein multihierarchisches Modell der Leber erstellt wird und anschließend deren Duchblutung simuliert wird.

Der Erfindung liegt die Aufgabe zugrunde, eine alternative Bestimmung einer in einem Gefäß vorherrschenden Strömungssituation zu ermöglichen.

Diese Aufgabe wird erfindungsgemäß gelöst durch ein Verfahren mit den Merkmalen des Anspruchs 1. Des Weiteren wird diese Aufgabe erfindungsgemäß gelöst durch eine Vorrichtung mit den Merkmalen des Anspruchs 13. Vorteilhafte und teils für sich erfinderische Ausführungsformen und Weiterentwicklungen der Erfindung sind in den Unteransprüchen und der nachfolgenden Beschreibung dargelegt.

Das erfindungsgemäße Verfahren dient zur Bestimmung einer Strömungssituation in einem Gefäß. Verfahrensgemäß wird dabei ein erster, Bildinformationen zu dem Gefäß - vorzugsweise einem Blutgefäß - enthaltender Bilddatensatz herangezogen. Vorzugsweise wird dieser (erste) Bilddatensatz dabei mittels eines gefäßabbildenden Bildgebungsverfahrens erzeugt und zur Durchführung des Verfahrens bereitgestellt. Anhand des ersten Bilddatensatzes wird daraufhin ein Gefäßbaum - d. h. vorzugsweise ein örtlicher Verlauf - des Gefäßes segmentiert. Anhand des ersten Bilddatensatzes oder eines zweiten Bilddatensatzes wird des Weiteren ein Organ segmentiert. Dem Organ wird daraufhin wenigstens ein Areal eines Parenchyms des Organs zugewiesen. Anschließend wird mittels einer Texturanalyse eine Textur dieses Areals des Organs ermittelt. In Abhängigkeit von der ermittelten Textur wird dem Areal des Organs eine Strömungseigenschaft zugewiesen. In Abhängigkeit von dem segmentierten Gefäßbaum und der dem Areal des segmentierten Organs zugewiesenen Strömungseigenschaft wird anschließend mittels einer numerischen Methode wenigstens ein Wert einer Messgröße bestimmt, die für die Strömungssituation innerhalb des Gefäßes charakteristisch ist. Vorzugsweise wird dabei für das Gefäß (oder: "an dem Gefäß") eine Strömungssimulation durchgeführt, in die der Gefäßbaum dieses Gefäßes und die dem Areal des Organs zugewiesene Strömungseigenschaft als Informationen einfließen. Als Ergebnis dieser Strömungssimulation wird dann vorzugsweise dieser wenigstens eine Wert der charakteristischen Messgröße ermittelt.

Unter dem Begriff "Segmentierung" oder "Segmentieren" wird hier und im Folgenden insbesondere eine Identifikation hinsichtlich ihrer Bildinformationen gleichartiger Bereiche innerhalb des Bilddatensatzes verstanden. Unter gleichartigen Bereichen werden dabei vorzugsweise jeweils spezifische Gewebearten, also bspw. ein Gefäß und/oder ein Organ verstanden. Im Rahmen der Segmentierung wird dabei vorzugsweise ein Bild oder Modell des Gefäßbaums (d. h. vorzugsweise des gesamten, in dem Bilddatensatz enthaltenen Gefäßes) erzeugt oder aus dem Bilddatensatz "extrahiert". Ebenso wird im Rahmen der entsprechenden Segmentierung des Organs dessen (vorzugsweise dreidimensionales Struktur-) Bild oder Modell aus dem ersten oder ggf. dem zweiten Bilddatensatz erzeugt. Mitunter liegt nach der jeweiligen Segmentierung ein vorzugsweise entsprechend zugewiesener Datensatz vor, der das Modell des Gefäßes bzw. des Organs enthält.

Bei dem ersten Bilddatensatz, der zur Segmentierung des Gefä-βes herangezogen wird, handelt es sich beispielsweise um einen unter Kontrastmittelgabe erzeugten Bilddatensatz, optional einen aus einem solchen Bilddatensatz bspw. mittels eines Subtraktionsverfahrens erzeugten Bilddatensatz. In Abhängigkeit von dem zur Segmentierung des Organs eingesetzten Verfahren, konkret dem eingesetzten Segmentierungsalgorithmus wird zur Segmentierung des Organs der gleiche, erste Bilddatensatz oder der insbesondere von diesem unterschiedliche zweite Bilddatensatz (bspw. ein Nativbild, ein Bild ohne Kontrastmittelgabe oder dergleichen) herangezogen.

"Charakteristisch" bedeutet hier und im Folgenden insbesondere, dass die Messgröße eine quantitative Information über die Strömungssituation, konkret einen Parameter (oder: Kenngröße) der Strömungssituation beinhaltet. Die Messgröße kann hierbei den entsprechenden Parameter der Strömungssituation unmittelbar angeben. Bei der Messgröße kann es sich aber auch um eine Größe handeln, die zu dem anzuzeigenden Parameter direkt oder indirekt proportional ist. Ferner kann die Messgröße mit dem anzuzeigenden Parameter auch in einer nicht-linearen, beispielsweise einer logarithmischen, exponentiellen oder polynomialen (also quadratischen, kubischen, etc.) Beziehung stehen.

Der Erfindung liegt die Erkenntnis zugrunde, dass eine numerische Berechnung des Werts der für die Strömungssituation innerhalb des Gefäßes charakteristischen Messgröße alleine anhand der auf den Gefäßbaum bezogenen Informationen nicht akzeptablen Ungenauigkeiten unterworfen ist. Denn ein Einfluss des Kapillarsystems des Gefäßes und/oder des Parenchyms des jeweiligen Organs, das durch das Gefäß versorgt wird, wird hierbei nicht berücksichtigt, da deren jeweilige Strukturen (bspw. der Durchmesser der Kapillaren) regelmäßig unterhalb der Auflösungsgrenze von herkömmlichen medizinischen Bildgebungsverfahren liegen. Eben diese Bereiche - d. h. das Kapillarsystem bzw. das angrenzende Parenchym - unterscheiden sich jedoch hinsichtlich ihrer Strömungseigenschaften regelmäßig deutlich von den Gefäßbereichen mit auflösbaren Durchmessern von insbesondere größer als 10 Mikrometern. Zudem beeinflussen diese Bereiche die Strömungssituation in den grö-βeren Gefäßbereichen zu einem nicht unerheblichen Maß. Aufgrund der erfindungsgemäßen Texturanalyse wird jedoch vorteilhafterweise ermöglicht, Rückschlüsse auf die tatsächlich (d. h. insbesondere patientenspezifisch) innerhalb des Areals des Organs vorliegenden Eigenschaften des Organs - wie z.B. dessen Dichte, dessen Gewebezusammensetzung, dessen Durchsetzung mit Kapillaren und dergleichen - zu ziehen. Diese Eigenschaften hängen dabei wiederum mit den Strömungseigenschaften innerhalb dieses Areals des Organs zusammen oder beeinflussen diese zumindest. Die Texturanalyse erlaubt es nun, auch für Bereiche mit nicht weiter auflösbaren Substrukturen anhand von (mathematischen oder numerischen) Analysen kleinster Unregelmäßigkeiten innerhalb dieser Bereiche die vorgenannten Rückschlüsse zu ziehen. Insbesondere kann somit nun vorteilhafterweise auch der Einfluss des Parenchyms des durch das Gefäß versorgten Organs bei der Bestimmung des Werts der charakteristischen Messgröße berücksichtigt werden. Aufgrund der Texturanalyse des Areals des Organs kann also die mit der Zuweisung der jeweiligen Strömungseigenschaft zu dem Areal des Parenchyms erreichbare Präzision der Bestimmung des Werts der Messgröße grundsätzlich, sowie auch gegenüber einer bloßen, pauschalen Annahme der Strömungseigenschaften des Parenchyms ohne genauere Kenntnis der tatsächlichen Eigenschaften des patientenspezifischen Parenchyms signifikant erhöht werden. Dadurch wird insgesamt eine hinreichend genaue Bestimmung der Strömungssituation, konkret des Werts der charakteristischen Messgröße innerhalb des Gefäßes auch ohne Eingriff an einer unzugänglichen Körperregion ermöglicht.

In einer bevorzugten Verfahrensvariante werden für den Gefäßbaum (vorzugsweise bereits im Rahmen der Segmentierung) geometrische Informationen, insbesondere ein örtlicher Verlauf eines Lumens des Gefäßes - insbesondere dessen Innendurchmesser oder Innenquerschnittsfläche - über den Gefäßbaum bestimmt. D. h. das Modell des Gefäßbaums enthält vorzugsweise Informationen, die zweckmäßigerweise für jede Stelle des Gefäßbaums das Lumen des Gefäßes wiedergeben. Die numerische Methode zur Bestimmung des Werts der charakteristischen Messgröße, d. h. insbesondere die Strömungssimulation wird dabei zweckmäßigerweise anhand der vorstehenden geometrischen Informationen durchgeführt.

In einer zweckmäßigen Verfahrensvariante wird als charakteristische Messgröße für die Strömungssituation innerhalb des Gefäßes ein Gefäßinnendruck, insbesondere ein Blutdruck herangezogen. Somit wird mittels der numerischen Methode, konkret der Strömungssimulation insbesondere der (vorzugsweise lokale) Wert des Gefäßinnendrucks bestimmt. Optional wird (zusätzlich oder alternativ zu dem Gefäßinnendruck) auch eine Strömungsgeschwindigkeit oder ein Volumenstrom insbesondere des innerhalb des Gefäßes strömenden Blutes als Messgröße herangezogen.

In einer weiteren zweckmäßigen Verfahrensvariante wird als die dem jeweiligen Areal des Organs zugewiesene Strömungseigenschaft ein Parameter (oder eine Kenngröße) mit einem zugeordneten (aktuellen) Wert herangezogen. Insbesondere wird als jeweils zugewiesene Strömungseigenschaft ein Strömungswiderstand (sowie dessen Wert), eine Strömungsgeschwindigkeit und/oder ein Blutfluss (insbesondere der Volumenstrom des Blutes) herangezogen. Diese Kenngrößen beeinflussen erkanntermaßen die in den übergeordneten, größeren (insbesondere auflösbaren) Gefäßbereichen vorherrschende Strömungssituation, bspw. den dortigen Blutdruck, Volumenstrom und dergleichen.

In einer zweckmäßigen Verfahrensvariante wird die dem Areal des Organs zugewiesene Strömungseigenschaft für die Strömungssimulation, d. h. für die Berechnung der charakteristischen Messgröße mittels der numerischen Methode als Randbedingung gesetzt. Diese Strömungseigenschaft stellt somit eine Größe dar, die insbesondere für einen an die in der Strömungssimulation berücksichtigte Struktur (hier insbesondere der Gefäßbaum, konkret dessen geometrisches Modell) angrenzenden Bereich eine insbesondere unveränderliche Eigenschaft, konkret einen unveränderlichen Wert einer für diese Eigenschaft charakteristischen Größe darstellt. Das heißt, dass die mittels der Texturanalyse ermittelte Strömungseigenschaft bei der Strömungssimulation für das Parenchym des Organs unveränderlich vorgegeben wird.

In einer weiteren zweckmäßigen Verfahrensvariante umfasst das dem Organ zugewiesene Areal (des Organparenchyms) einen Bereich, für den eine Auflösungsgrenze für das Gefäß, insbesondere für dessen Gefäßbaum unterschritten wird. D. h. das Areal des Organs wird also eben für diesen, unter der Auflösungsgrenze liegenden Bereich des Gefäßbaums ermittelt. Somit wird auch die Texturanalyse vorteilhafterweise (und insbesondere "nur") für die Bereiche durchgeführt, für die keine Äste oder Kapillaren des Gefäßbaums mehr aufgelöst werden können und für die somit auch keine geometrischen Informationen für die Strömungssimulationen bestimmt werden können. Diese Bereiche, konkret deren nach der Texturanalyse zugewiesene Strömungseigenschaften finden dann wie vorstehend beschrieben insbesondere als Randbedingungen Einzug in die vorstehend beschriebene Strömungssimulation.

In einer besonders zweckmäßigen Verfahrensvariante wird das Parenchym des Organs dabei insbesondere in eine Mehrzahl - d. h. zwei oder mehr - Areale unterteilt, die vorzugsweise jeweils durch einen Gefäßast des Gefäßbaums versorgt werden. Insbesondere wird ein solches Areal dabei von jeweils einem arteriellen und einem venösen Ast jeweils eines (arteriellen bzw. venösen) Gefäßes versorgt. Für jedes dieser mehreren Areale wird anschließend insbesondere jeweils eine Texturanalyse durchgeführt. Entsprechend wird anschließend jedem der Areale auch jeweils wenigstens eine Strömungseigenschaft zugewiesen. Durch die Aufteilung des Organs in mehrere Areale kann vorteilhafterweise die Präzision der bei der Strömungssituation verwendeten Randbedingungen gesteigert werden. So können insbesondere bei Organen, die über ihr gesamtes Volumen verteilt häufig verschiedene Gewebeeigenschaften (die sich insbesondere in der jeweiligen Textur niederschlagen) - ggf. auch krankheitsbedingt - aufweisen können, durch mehrere Strömungseigenschaften, die für den jeweiligen Bereich bzw. das jeweilige Areal die dort vorherrschenden Eigenschaften vergleichsweise präzise wiedergeben, insgesamt die Präzision und die Auflösung der Strömungseigenschaften innerhalb des Organs und somit auch die Präzision der Strömungssimulation selbst gesteigert werden. Insbesondere kann bei der Strömungssimulation vorteilhafterweise jedem insbesondere gerade noch auflösbaren Gefäßast die Strömungseigenschaft des angrenzenden Parenchymareals als (lokale) Randbedingung zugewiesen werden. In einer alternativen oder optional zusätzlichen Verfahrensvariante wird jedoch das gesamte Organ durch ein einziges Areal dargestellt, und somit dem gesamten Organ eine einheitliche Strömungseigenschaft (bspw. ein Strömungswiderstand für das gesamte Organ) zugewiesen. Dies ist beispielsweise bei vergleichsweise kleinen Organen und/oder Organen zweckmäßig, die regelmäßig keine oder lediglich vernachlässigbare Abweichungen in ihren Texturen über das gesamte Organ aufweisen. In diesem Fall kann dann vorteilhafterweise Analyseaufwand im Rahmen der Texturanalyse eingespart werden.

In einer weiteren zweckmäßigen Verfahrensvariante werden zumindest der Gefäßbaum und der wenigstens eine, zugeordnete Wert der charakteristischen Messgröße grafisch - vorzugsweise auf einer Anzeige einer das Verfahren ausführenden Modalität oder Vorrichtung - dargestellt. Beispielsweise werden dabei für unterschiedliche Bereiche des Gefäßbaums die jeweiligen Werte der charakteristischen Messgröße angezeigt, indem insbesondere der jeweilige Wert dem entsprechenden Bereich (bspw. mittels einer Bezugslinie) zugeordnet wird. Insbesondere bei der Darstellung von Blutdruckwerten erfolgt dabei alternativ oder optional zusätzlich auch eine qualitative Darstellung der Verteilung der einzelnen Blutdruckwerte über den Gefäßbaum. Vorzugsweise wird der Gefäßbaum dabei farblich in Abhängigkeit von dem jeweiligen Blutdruckwert kodiert dargestellt. Die Farben sind dabei vorzugsweise in einem Ampelschema wenigstens einem Normal- und einem Überdruck zugeordnet, bspw. grün für Normaldruck und rot für Überdruck. So kann medizinisches Fachpersonal auf einfache Weise und schnell Bereiche des Gefäßbaums identifizieren, in denen ungünstige oder gar kritische Blutdruckwerte vorliegen. Optional ist auch einem Unterdruck eine Farbe zugeordnet.

In einer zweckmäßigen Verfahrensvariante erfolgt die Zuordnung der jeweiligen Strömungseigenschaft zu dem oder dem jeweiligen Areal des Organs auf Basis eines maschinellen Lernverfahrens. Das heißt, dass die Texturanalyse vorzugsweise mittels eines "selbstlernenden" Analysealgorithmus implementiert ist, der insbesondere anhand von empirisch ermittelten Daten "trainiert" wird. Als empirische Daten werden bspw. Vergleichsdaten herangezogen, bei denen mittels herkömmlichen (insbesondere invasiven) Messverfahren der Blutdruck innerhalb eines Gefäßes bestimmt wurde. Auf Basis dieser Messwerte wird den jeweiligen, mittels der Texturanalyse zu analysierenden Arealen des Organs dann die jeweilige Strömungseigenschaft derart zugewiesen, dass die nachfolgende Strömungssimulation des Gefäßbaums, konkret deren Ergebnis, an das tatsächlich gemessene Ergebnis angenähert, vorzugsweise mit diesem zur Deckung gebracht wird. Durch ein solches, wiederholtes "reverse analyzing" wird dem Analysealgorithmus antrainiert, welche Strömungseigenschaften spezifischen Texturen zuzuweisen sind. Alternativ wird im Rahmen der Texturanalyse zur Zuweisung der jeweiligen Strömungseigenschaft auf eine "Lookup-Tabelle" zurückgegriffen, in der empirisch ermittelte Strömungseigenschaften für vorbekannte Texturen hinterlegt sind. Im Rahmen der Texturanalyse werden die ermittelten Texturen mit den vorbekannten Texturen (bspw. über eine Mustererkennung) verglichen und die entsprechende Strömungseigenschaft aus der Tabelle ausgelesen. In einer weiteren optionalen Variante ist für die Texturanalyse ein Modell der Strömungseigenschaften in Abhängigkeit von der Textur implementiert. Somit werden in diesem Fall die jeweiligen Strömungseigenschaften mittels eines numerischen Verfahrens anhand der Textur bestimmt.

In einer besonders bevorzugten Verfahrensvariante wird zur Erzeugung insbesondere des ersten, optional aber auch des zweiten Bilddatensatzes als bildgebende Modalität ein sogenannter direkt-konvertierender Computertomograph (CT) herangezogen. Dieser weist vorteilhafterweise eine besonders hohe Auflösung auf, so dass auch noch vergleichsweise kleine Gefäßäste (d. h. Gefäße mit vergleichsweise kleinem Durchmesser) dargestellt oder aufgelöst werden können. Alternativ kommen auch Bildgebungsverfahren wie zum Beispiel (ggf. Kontrastmittel-unterstützte oder flusssensitive) Magnetresonanztomographie (MRT), Positronen-Emissions-Tomographie (PET), (Doppler-)Ultraschall, Angiographie und dergleichen zum Einsatz.

Optional wird für die Texturanalyse ebenfalls der erste Bilddatensatz herangezogen. In einer zweckmäßigen Verfahrensvariante wird aber für die Texturanalyse ein zweiter oder ggf. ein dritter Bilddatensatz herangezogen. Dieser zweite oder dritte Bilddatensatz wird dabei optional von der gleichen bildgebenden Modalität wie der erste Bilddatensatz bereitgestellt. In diesem Fall handelt es sich bei dem zweiten oder dritten Bilddatensatz bspw. um ein Nativbild, d. h. um ein Bild bzw. einen Bilddatensatz, der noch nicht durch die Anwendung eines Bilddatenverarbeitungsalgorithmus verändert wurde. Alternativ handelt es sich bei dem zweiten oder dritten Bilddatensatz um einen solchen, der von einer anderen bildgebenden Modalität, d. h. auf Basis eines von dem für den ersten Bilddatensatz unterschiedlichen bildgebenden Verfahrens erzeugt wurde. Für den Fall, dass für die Texturanalyse der zweite oder dritte Bilddatensatz herangezogen wird, wird dieser zunächst mit dem ersten bzw. dem zweiten Bilddatensatz zur Deckung gebracht, so dass das jeweilige (anhand des ersten oder zweiten Bilddatensatzes ermittelte) Areal des Organs auf dem korrespondierenden Bereich des zweiten bzw. dritten Bilddatensatzes abgebildet wird.

In einer zweckmäßigen Verfahrensvariante werden zur Bestimmung des Werts der charakteristischen Messgröße mittels der numerischen Methode, d. h. in der Strömungssimulation auch zusätzliche Hilfsinformationen herangezogen. Bei diesen Hilfsinformationen handelt es sich beispielsweise um Gradienten einer Kontrastmittelkonzentration (zeitliche und/oder örtliche Gradienten), sogenannte Time to Peak-Informationen bei einer Kontrastmittelgabe und dergleichen. Zusätzlich werden als Hilfsinformationen auch patientenspezifische Daten wie z. B. Größe und/oder Gewicht des Organs, des Patienten selbst, BMI, Alter, Geschlecht, Spirometriedaten, Diffusionsdaten, Laborwerte (insbesondere einer Blutuntersuchung) des Patienten und dergleichen herangezogen. Dadurch kann das Ergebnis der Strömungssimulation verbessert und/oder der Rechenaufwand verringert werden.

In einer weiteren zweckmäßigen Verfahrensvariante werden (vorzugsweise nachfolgend zu einem ersten Simulationsdurchlauf nach vorstehend beschriebener Art zur Bestimmung des Ist-Zustands) der Gefäßbaum und/oder das oder das jeweilige Areal das Organ verändert. Insbesondere wird dabei das jeweilige Modell - d. h. der örtliche, insbesondere geometrische Verlauf des Gefäßbaums oder bspw. die Struktur des Organs - verändert. Anschließend wird der veränderte Gefäßbaum bzw. das veränderte Organ bei der Bestimmung des Werts der charakteristischen Messgröße - d. h. bei der Strömungssimulation - berücksichtigt. Insbesondere wird dabei in Abhängigkeit von der Veränderung des jeweiligen Areals des Organs diesem eine (insbesondere entsprechend veränderte) Strömungseigenschaft zugewiesen. Anschließend wird die Strömungssimulation in Abhängigkeit von dem veränderten Gefäßbaum bzw. der jeweiligen, veränderten Strömungseigenschaft durchgeführt und der jeweilige Wert der charakteristischen Messgröße bestimmt. Dadurch wird es vorteilhafterweise ermöglicht, die Ergebnisse eines geplanten invasiven Eingriffs, der zu der oben genannten Veränderung des Gefäßbaums bzw. des Organs führen kann (insbesondere soll), abzuschätzen. Insbesondere wird dazu ein Vergleich der simulierten Strömungssituationen vor und nach der jeweiligen Veränderung durchgeführt. Bei der vorgenannten Veränderung handelt es sich bspw. um eine geplante Resektion von Gewebe des Organs und/oder eines Gefäßastes, um das Einsetzen eines Stents in einen Gefäßast oder dergleichen. Das vorstehend beschriebene Verfahren kann somit vorteilhafterweise nicht nur zur Ermittlung des Istzustands der Strömungssituation in einem Gefäß sondern auch zur Planung, insbesondere zur Abschätzung eines Ergebnisses eines invasiven Eingriffs herangezogen werden.

Die numerische Methode bzw. die Strömungssimulation basiert bspw. auf Näherungslösungen für einzelne Abschnitte des Gefäßbaums, die insbesondere Strömungsgesetze wie nach Hagen-Poiseuille, Bernoulli etc. berücksichtigen, auf Lösungen von Differentialgleichungen (Navier-Stokes, Euler etc.), auf maschinellen Lernverfahren, Widerstandsberechnung nach Art der Kirchhoffschen Regeln und dergleichen.

Die erfindungsgemäße Vorrichtung dient zur Bestimmung der Strömungssituation in dem Gefäß. Die Vorrichtung umfasst dazu einen Controller - auch als Recheneinheit bezeichnet -, der zur Durchführung des vorstehend beschriebenen Verfahrens eingerichtet und vorgesehen ist. Besonders bevorzugt ist die Vorrichtung, insbesondere der Controller mit wenigstens einer bildgebenden Modalität datenübertragungstechnisch verknüpft. Optional handelt es sich bei der Vorrichtung insbesondere um die Bildverarbeitungseinheit der jeweiligen bildgebenden Modalität selbst. Alternativ ist die Vorrichtung wenigstens mit einem Netzwerk (bspw. einem Krankenhausnetzwerk) verbunden, in dem die vorstehen beschriebenen Bilddatensätze hinterlegt sind. Der Controller ist dabei dazu eingerichtet, aus dem vorstehend beschriebenen ersten Bilddatensatz den Gefäßbaum zu segmentieren. Des Weiteren ist der Controller dazu eingerichtet, anhand des ersten Bilddatensatzes oder des zweiten Bilddatensatzes das jeweilige (insbesondere das zu untersuchende) Organ zu segmentieren. Ferner ist der Controller dazu eingerichtet, dem Organ ein oder mehrere Areale seines Parenchyms zuzuweisen und für jedes dieser Areale jeweils eine Texturanalyse zur Bestimmung der jeweiligen Textur durchzuführen. Außerdem ist Controller dazu eingerichtet, dem jeweiligen Areal in Abhängigkeit von der ermittelten Textur eine Strömungseigenschaft zuzuweisen und anschließend in Abhängigkeit von dem Gefäßbaum und der jeweiligen Strömungseigenschaft die numerische Methode, d. h. insbesondere die Strömungssimulation zur Bestimmung des Werts der für die Strömungssituation innerhalb des Gefäßes charakteristischen Messgröße durchzuführen.

Die erfindungsgemäße Vorrichtung teilt somit alle Vorteile des vorstehend beschriebenen, erfindungsgemäßen Verfahrens.

Die Konjunktion "und/oder" ist hier und im Folgenden derart zu verstehen, dass die mittels dieser Konjunktion verknüpften Merkmale oder Begriffe sowohl gemeinsam als auch als Alternativen zueinander ausgebildet sein bzw. auftreten können.

Nachfolgend werden Ausführungsbeispiele der Erfindung anhand einer Zeichnung näher erläutert. Darin zeigen:
- FIG 1: in einem schematischen Ablaufdiagramm den Ablauf eines Verfahrens zur Bestimmung einer Strömungssituation in einem Gefäß,
- FIG 2: in schematischer Draufsicht vier Schnittbilder durch ein Organ, und
- FIG 3: in einer schematischen Darstellung eine Vorrichtung zur Durchführung des Verfahrens gemäß FIG 1.

Einander entsprechende Teile und Größen sind in allen Figuren stets mit gleichen Bezugszeichen versehen.

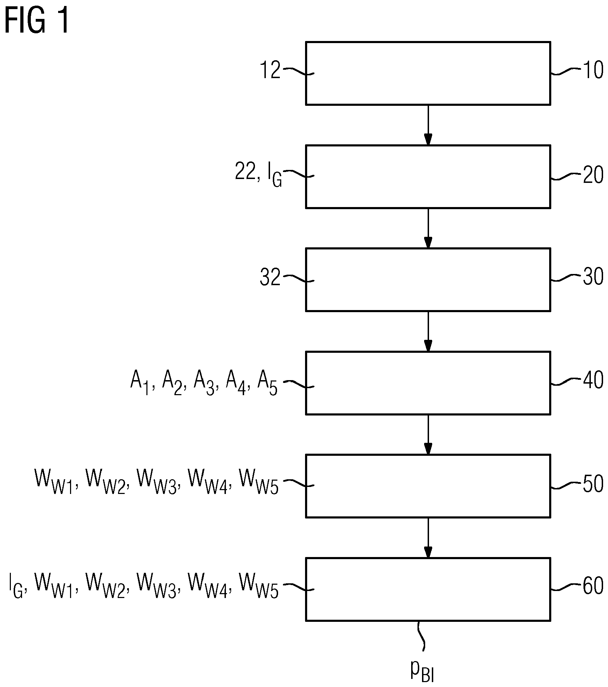

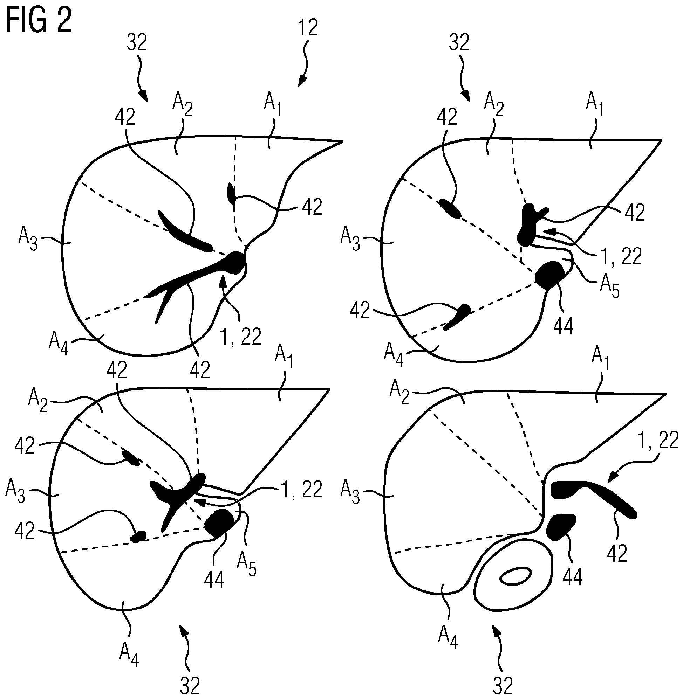

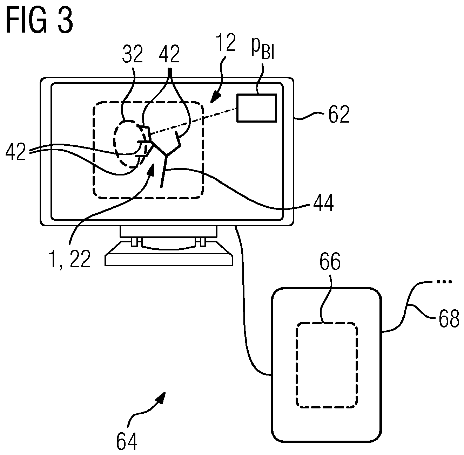

In FIG 1 ist in einem schematischen Ablaufdiagramm der Ablauf eines Verfahrens zur Bestimmung einer Strömungssituation in einem Gefäß 1 (vgl. FIG 2 und FIG 3) dargestellt. In einem ersten Verfahrensschritt 10 wird zunächst ein erster Bilddatensatz 12, der Bildinformationen zu dem Gefäß 1 enthält, bereitgestellt. Konkret wird dieser erste Bilddatensatz 12 aus einem Speicher ausgelesen, d. h. geladen. In einem optionalen Ausführungsbeispiel wird der erste Bilddatensatz 12 im Rahmen des ersten Verfahrensschritts 10 mittels einer bildgebenden Modalität, konkret einem Computertomographen, erzeugt. In einem zweiten Verfahrensschritt 20 wird anhand des ersten Bilddatensatzes 12 ein Gefäßbaum 22 des Gefäßes 1 segmentiert. Der Gefäßbaum 22 stellt dabei den örtlichen, konkret geometrischen Verlauf des Gefäßes 1 innerhalb des von dem Bilddatensatz 12 beschriebenen Bildes - vorzugsweise ein volumetrisches Bild, das ggf. in mehrere Schnittbilder (s. FIG 2) unterteilt ist - dar. Im Rahmen der Segmentierung, d. h. des zweiten Verfahrensschrittes 20, werden dabei auch geometrische Informationen I_{G} zu dem Gefäßbaum 22 ermittelt. Diese geometrischen Informationen I_{G} geben dabei konkret den örtlichen Verlauf eines Lumens - d. h. des Werts des Innendurchmessers oder der Innenquerschnittsfläche - des Gefäßes 1 entlang des Gefäßbaums 22 wieder. In einem dritten Verfahrensschritt 30 wird anhand des ersten Bilddatensatzes 12 ein Organ - im vorliegenden Ausführungsbeispiel die Leber 32 - segmentiert. D. h. es werden in dem dritten Verfahrensschritt 30 die Anteile des ersten Bilddatensatzes 12 bestimmt, die die Leber 32 abbilden. Dabei wird in vergleichbarer Weise zu dem zweiten Verfahrensschritt 20 ein Strukturbild des Organs, d. h. der Leber 32 erzeugt.

In einem vierten Verfahrensschritt 40 wird für das Parenchym, d. h. für das funktionale Gewebe der Leber 32 eine Anzahl von Arealen A₁-A₅ (vgl. FIG 2) bestimmt und der Leber 32 zugewiesen. Mit anderen Worten wird die Leber 32 in diese Areale A₁-A₅ unterteilt. Jedes dieser Areale wird dabei von einem Gefäßast 42 des Gefäßbaums 22 "versorgt". Jeder Gefäßast 42 stellt dabei einen von einem Hauptabschnitt oder Stamm 44 des Gefäßes 1 abgezweigten Abschnitt dar.

In einem nachfolgenden Verfahrensschritt 50 wird für jedes Areal A₁-A₅ der Leber 32 eine Texturanalyse durchgeführt. Konkret wird für jedes Areal A₁-A₅ eine (mathematische) Auswertung des ersten Bilddatensatzes 12 durchgeführt, mittels derer Unregelmäßigkeiten innerhalb des das jeweilige Areal A₁-A₅ bildenden Bildbereichs erkannt sowie die Dichte des dortigen Gewebes analysiert, konkret ermittelt wird. Als Ergebnis dieser Texturanalyse wird jedem der Areale A₁-A₅ eine Textur zugewiesen. In Abhängigkeit dieser Textur wird anschließend jedem der Areale A₁-A₅ eine Strömungseigenschaft, konkret ein aktueller Wert eines Strömungswiderstands (im Folgenden als Widerstandswert W_{W1}-W_{W5} bezeichnet) zugewiesen.

In einem weiteren Verfahrensschritt 60 wird ein Wert einer für die Strömungssituation innerhalb des Gefäßes 1 charakteristischen Messgröße bestimmt. Bei dieser charakteristischen Messgröße handelt es sich konkret um den Blutdruck innerhalb des Gefäßes 1. Der ermittelte Wert wird hier und im Folgenden als Blutdruckistwert p_{BI} bezeichnet. Zur Bestimmung des Blutdruckistwerts p_{BI} wird im Rahmen des Verfahrensschritts 60 eine numerische Methode angewendet, konkret eine Strömungssimulation durchgeführt. Die Strömungssimulation wird dabei für den Gefäßbaum 22 - d. h. für die in dem ersten Bilddatensatz 12 aufgelösten Teile des Gefäßes 1 - in Abhängigkeit von den geometrischen Informationen I_{G} durchgeführt. Als Randbedingungen für die unterhalb der Auflösungsgrenze des ersten Bilddatensatzes 12 liegenden Bereiche des Gefäßbaums 22 sowie für den Einfluss des Parenchyms der Leber 32 werden die Widerstandswerte W_{W1}-W_{W5} in der Strömungssimulation verwendet. Die Strömungssimulation wird dabei auf Basis der geometrischen Informationen I_{G} und der als Randbedingung gesetzten Widerstandwerte W_{W1}-W_{W5} durch Lösung von Navier-Stokes-Gleichungen durchgeführt.

In einem nachfolgenden, nicht näher dargestellten Verfahrensschritt, wird der Blutdruckistwert p_{BI} zur Information von medizinischem Fachpersonal ausgegeben. Konkret wird der Blutdruckistwert p_{BI} dabei über eine Anzeigevorrichtung, beispielsweise einen Monitor 62 (vgl. FIG 3), dargestellt.

In FIG 3 ist eine Vorrichtung zur Durchführung des vorstehend beschriebenen Verfahrens dargestellt. Bei der Vorrichtung handelt es sich konkret um einen Computer 64, der den Monitor 62 umfasst. Der Computer 64 umfasst außerdem auch einen Controller, konkret einen Mikroprozessor 66, in dem die Funktionalität zur Durchführung des vorstehend beschriebenen Verfahrens als ausführbare Software implementiert ist. Der Computer 64 ist dabei über eine Datenleitung 68 mit einem Krankenhausnetzwerk, in dem der Bilddatensatz 12 in einer Datenbank abgespeichert ist, verbunden. In einem optionalen Ausführungsbeispiel ist der Computer 64 mittels der Datenleitung 68 direkt mit der bildgebenden Modalität verbunden.

In FIG 3 ist auf dem Monitor 62 beispielhaft eine Abbildung des Ergebnisses der Strömungssituation dargestellt. Der Blutdruckistwert p_{BI} ist dabei einem Abschnitt des Gefäßbaums 22 mittels einer Bezugslinie zugewiesen. Konkret ist in aus Gründen der Übersichtlichkeit nicht näher dargestellter Weise verschiedenen Abschnitten des Gefäßbaums 22 jeweils ein Blutdruckistwert p_{BI} zugewiesen.

In einer alternativen oder optional zusätzlich wählbaren Anzeigevariante ist der Gefäßbaum 22 farblich kodiert in Abhängigkeit von dem jeweils vorherrschenden Blutdruckistwert p_{BI} dargestellt. So ist ein Bereich, in dem der Blutdruckistwert p_{BI} einem Normaldruck entspricht, grün markiert. Ein anderer Bereich des Gefäßbaums 22, in dem der Blutdruckistwert p_{BI} den Normaldruck überschreitet, ist dabei beispielsweise rot markiert, sodass das medizinische Fachpersonal auf einfache Weise auf diesen Bereich aufmerksam gemacht werden kann.

In einem nicht näher dargestellten optionalen Ausführungsbeispiel werden die Verfahrensschritte 30, 40 und 50 anhand eines zweiten Bilddatensatzes durchgeführt. Dieser zweite Bilddatensatz ist beispielsweise ein Nativbild, aus dem der erste Bilddatensatz beispielsweise durch ein Subtraktionsverfahren generiert wurde. Alternativ handelt es sich bei dem zweiten Bilddatensatz um einen Bilddatensatz, der mittels einer anderen bildgebenden Modalität als dem für den ersten Bilddatensatz 12 herangezogenen Computertomographen erzeugt wurde.

## Patentansprüche

1. Verfahren zur Bestimmung einer Strömungssituation in einem Gefäß (1), wobei verfahrensgemäß
- ein erster, Bildinformationen zu dem Gefäß (1) enthaltender Bilddatensatz (12) herangezogen wird,
- anhand des ersten Bilddatensatzes (12) ein Gefäßbaum (22) des Gefäßes (1) segmentiert wird,
- anhand des ersten Bilddatensatzes (12) oder eines zweiten Bilddatensatzes ein Organ (32) segmentiert wird,
- dem Organ (32) wenigstens ein Areal (A₁-A₅) eines Parenchyms des Organs (32) zugewiesen wird, **dadurch gekennzeichnet, dass**
- mittels einer Texturanalyse welche eine mathematische oder numerische Analyse kleinster Unregelmäßigkeiten in dem Areal umfasst, eine Textur des Areals (A₁-A₅) des Organs (32) ermittelt wird,
- in Abhängigkeit von der Textur dem Areal (A₁-A₅) des Organs (32) eine Strömungseigenschaft (W_{W1-}W_{W5}) zugewiesen wird,
- in Abhängigkeit von dem Gefäßbaum (22) und der dem Areal (A₁-A₅) des Organs (23) zugewiesenen Strömungseigenschaft (W_{W1-}W_{W5}) mittels einer numerischen Methode wenigstens ein Wert (p_{BI}) einer für die Strömungssituation innerhalb des Gefäßes (1) charakteristischen Messgröße bestimmt wird.

2. Verfahren nach Anspruch 1,
wobei für den Gefäßbaum (22) geometrische Informationen (I_{G}), insbesondere ein örtlicher Verlauf eines Lumens des Gefäßes (1) über den Gefäßbaum (22) bestimmt werden, und wobei die numerische Methode auf Basis der geometrischen Informationen (I_{G}) durchgeführt wird.

3. Verfahren nach Anspruch 1 oder 2,
wobei als charakteristische Messgröße ein Gefäßinnendruck, insbesondere ein Blutdruck herangezogen wird.

4. Verfahren nach einem der Ansprüche 1 bis 3,
wobei als dem Areal (A₁-A₅) zugewiesene Strömungseigenschaft ein Wert (W_{W1-}W_{W5}) eines Strömungswiderstands, einer Strömungsgeschwindigkeit und/oder eines Blutflusses herangezogen wird.

5. Verfahren nach einem der Ansprüche 1 bis 4,
wobei die dem Areal (A₁-A₅) des Organs (32) zugewiesene Strömungseigenschaft (W_{W1}-W_{W5}) im Rahmen der numerischen Methode als Randbedingung herangezogen wird.

6. Verfahren nach einem der Ansprüche 1 bis 5,
wobei das Areal (A₁-A₅) des Organs (32) für einen Bereich ermittelt wird, für den eine Auflösungsgrenze für den Gefäßbaum (22) unterschritten wird.

7. Verfahren nach einem der Ansprüche 1 bis 6,
wobei das Parenchym des Organs (32) in eine Mehrzahl von Arealen (A₁-A₅) unterteilt wird, und wobei die Texturanalyse für jedes der Areale (A₁-A₅) des Parenchyms durchgeführt wird.

8. Verfahren nach einem der Ansprüche 1 bis 7,
wobei der Gefäßbaum (22) und der wenigstens eine zugeordnete Wert (p_{BI}) der charakteristischen Messgröße grafisch dargestellt wird.

9. Verfahren nach einem der Ansprüche 1 bis 8,
wobei die Zuordnung der jeweiligen Strömungseigenschaft (W_{W1-}W_{W5}) zu dem oder dem jeweiligen Areal (A₁-A₅) des Organs (32) auf Basis eines maschinellen Lernverfahrens erfolgt.

10. Verfahren nach einem der Ansprüche 1 bis 9,
wobei für die Texturanalyse ein zweiter oder dritter Bilddatensatz herangezogen wird.

11. Verfahren nach einem der Ansprüche 1 bis 10,
wobei zur Bestimmung des Werts (p_{BI}) der charakteristischen Messgröße zusätzliche Hilfsinformationen herangezogen werden.

12. Verfahren nach einem der Ansprüche 1 bis 11,
wobei der Gefäßbaum (22) und/oder das oder das jeweilige Areal (A₁-A₅) des Organs (32) verändert werden und wobei in Abhängigkeit von dem veränderten Gefäßbaum (22) und der dem veränderten Areal (A₁-A₅) des Organs (23) zugewiesenen Strömungseigenschaft (W_{W1-}W_{W5}) der Wert (p_{BI}) der charakteristischen Messgröße bestimmt wird.

13. Vorrichtung (64) zur Bestimmung einer Strömungssituation in einem Gefäß (1), mit einem Controller (66) der zur Durchführung des Verfahrens nach einem der Ansprüche 1 bis 12 eingerichtet ist.

## Claims

1. Method for determining a flow situation in a vessel (1),
wherein according to the method
- a first image data set (12) containing image information relating to the vessel (1) is used,
- a vascular tree (22) of the vessel (1) is segmented on the basis of the first image data set (12),
- an organ (32) is segmented on the basis of the first image data set (12) or of a second image data set,
- the organ (32) is assigned at least one area (A₁-A₅) of a parenchyma of the organ (32), **characterised in that**
- by means of texture analysis which comprises a mathematical or numerical analysis of minute irregularities within the area, a texture of the area (A₁-A₅) of the organ (32) is determined,
- depending on the texture, the area (A₁-A₅) of the organ (32) is assigned a flow characteristic (W_{W1}-W_{W5}),
- depending on the vascular tree (22) and the flow characteristic (W_{W1}-W_{W5}) assigned to the area (A₁-A₅) of the organ (23), a value (p_{BI}) of a measured variable characteristic of the flow situation within the vessel (1) is determined by means of a numerical method.

2. Method according to claim 1,
wherein, for the vascular tree (22), geometric information (I_{G}) about the vascular tree (22), in particular a local path of a lumen of the vessel (1), is determined, and wherein the numerical method is carried out on the basis of the geometric information (I_{G}).

3. Method according to claim 1 or 2,
wherein an intravascular pressure, in particular a blood pressure, is used as a characteristic measured variable.

4. Method according to one of claims 1 to 3,
wherein a value (W_{W1}-W_{W5}) of a flow resistance, of a flow velocity and/or of a blood flow is used as the flow characteristic assigned to the area (A₁-A₅).

5. Method according to one of claims 1 to 4,
wherein the flow characteristic (W_{W1}-W_{W5}) assigned to the area (A₁-A₅) of the organ (32) is used as a boundary condition within the numerical method.

6. Method according to one of claims 1 to 5,
wherein the area (A₁-A₅) of the organ (32) is determined for a region for which the resolution is below a resolution limit for the vascular tree (22).

7. Method according to one of claims 1 to 6,
wherein the parenchyma of the organ (32) is sub-divided into a plurality of areas (A₁-A₅), and wherein texture analysis is carried out for each of the areas (A₁-A₅) of the parenchyma.

8. Method according to one of claims 1 to 7,
wherein the vascular tree (22) and the at least one assigned value (p_{BI}) of the characteristic measured variable is visually displayed.

9. Method according to one of claims 1 to 8,
wherein the flow characteristic (W_{W1}-W_{W5}) is assigned to the organ (32) or the respective area (A₁-A₅) of the organ (32) on the basis of a machine learning process.

10. Method according to one of claims 1 to 9,
wherein a second or third image data set is used for texture analysis.

11. Method according to one of claims 1 to 10,
wherein additional auxiliary information is used to determine the value (p_{BI}) of the characteristic measured variable.

12. Method according to one of claims 1 to 11,
wherein the vascular tree (22) and/or the organ (32) or the respective area (A₁-A₅) of the organ (32) are modified and wherein the value (p_{BI}) of the characteristic measured variable is determined according to the modified vascular tree (22) and the flow characteristic (W_{W1}-W_{W5}) assigned to the modified area (A₁-A₅) of the organ (23).

13. Device (64) for determining a flow situation in a vessel (1), comprising a controller (66) designed to carry out the method according to one of claims 1 to 12.

## Revendications

1. Procédé de détermination d'une situation d'écoulement dans un vaisseau (1), dans lequel suivant le procédé
- on tire parti d'un premier ensemble (12) de données d'image contenant des informations d'image sur le vaisseau (1),
- à l'aide du premier ensemble (12) de données d'image, on segmente un arbre (22) du vaisseau (1),
- à l'aide du premier ensemble (12) de données d'image ou d'un deuxième ensemble de données d'image, on segmente un organe (32),
- on affecte, à l'organe (32), au moins une aire (A₁-A₅) d'un parenchyme de l'organe (32),
**caractérisé en ce que**
- au moyen d'une analyse de texture, qui comprend une analyse mathématique ou numérique des irrégularités les plus petites dans l'aire, on détermine une texture de l'aire (A₁-A₅) de l'organe (32),
- on affecte une propriété (W_{W1}-W_{W5}) d'écoulement à l'aire (A₁-A₅) de l'organe (32), en fonction de la texture,
- on détermine au moins une valeur (p_{BI}) d'une grandeur de mesure caractéristique de la situation d'écoulement au sein du vaisseau (1) au moyen d'une méthode numérique, en fonction de l'arbre (22) du vaisseau et de la propriété (W_{W1}-W_{W5}) d'écoulement affectée à l'aire (A₁-A₅) de l'organe (23) .

2. Procédé suivant la revendication 1,
dans lequel on détermine, pour l'arbre (22) du vaisseau, des informations (I_{G}) géométriques, en particulier un tracé dans l'espace d'un lumen du vaisseau (1) sur l'arbre (22) du vaisseau, et dans lequel on effectue la méthode numérique sur la base des informations (I_{G}) géométriques.

3. Procédé suivant la revendication 1 ou 2,
dans lequel on tire parti, comme grandeur de mesure caractéristique, d'une pression à l'intérieur du vaisseau, en particulier d'une pression sanguine.

4. Procédé suivant l'une des revendications 1 à 3,
dans lequel on tire parti, comme propriété d'écoulement affectée à l'aire (A₁-A₅), d'une valeur (W_{W1}-W_{W5}) d'une résistance à l'écoulement, d'une vitesse à l'écoulement et/ou d'un flux sanguin.

5. Procédé suivant l'une des revendications 1 à 4,
dans lequel on tire parti, comme conditions aux limites dans le cadre de la méthode numérique, de la propriété (W_{W1}-W_{W5}) d'écoulement affectée à l'aire (A₁-A₅) de l'organe (32).

6. Procédé suivant l'une des revendications 1 à 5,
dans lequel on détermine l'aire (A₁-A₅) de l'organe (32) pour une partie, pour laquelle à une limite de résolution, pour l'arbre (22) du vaisseau est dépassée par le bas.

7. Procédé suivant l'une des revendications 1 à 6,
dans lequel on subdivise le parenchyme de l'organe (32) en une pluralités d'aires (A₁-A₅), et dans lequel on effectue l'analyse de texture pour chacune des aires (A₁-A₅) du parenchyme.

8. Procédé suivant l'une des revendications 1 à 7,
dans lequel on représente graphiquement l'arbre (22) du vaisseau et la au moins une valeur (p_{BI}) associée de la grandeur de mesure caractéristique.

9. Procédé suivant l'une des revendications 1 à 8,
dans lequel l'affectation de la propriété (W_{W1}-W_{W5}) respective d'écoulement à la ou aux aires (A₁-A₅) respectives de l'organe (32) s'effectue sur la base d'un procédé d'apprentissage automatique.

10. Procédé suivant l'une des revendications 1 à 9,
dans lequel on tire parti d'un deuxième ou d'un troisième ensemble de données d'image pour l'analyse de textures.

11. Procédé suivant l'une des revendications 1 à 10,
dans lequel on tire parti d'informations auxiliaires supplémentaires pour la détermination de la valeur (p_{BI}) de la grandeur de mesure caractéristique.

12. Procédé suivant l'une des revendications 1 à 11,
dans lequel on modifie l'arbre (22) du vaisseau et/ou l'aire ou les aires (A₁-A₅) respectives de l'organe (32), et dans lequel on détermine la valeur (p_{BI}) de la grandeur de mesure caractéristique en fonction de l'arbre (22) de vaisseau modifié et de la propriété (W_{W1}-W_{W5}) d'écoulement affectée à l'aire (A₁-A₅) modifiée de l'organe (23) .

13. Installation (64) de détermination d'une situation d'écoulement dans un vaisseau (1), comprenant une unité (66) de commande, qui est agencée pour effectuer le procédé suivant l'une des revendications 1 à 12.
